# EUROPEAN PATENT APPLICATION

(11) **EP 4 825 307 A1**
(43) Date of publication of application: **21.10.2026**
(21) Application number: 24903999.1
(22) Date of filing: 11.11.2024
(51) Int. Cl.: A61B 5/00, A61B 5/318, A61B 5/1455, A61B 5/021, A61B 5/024, A61B 5/08

(54) **BIOMETRIC DATA MONITORING SYSTEM HAVING ALARM FUNCTION**

(30) Priority: 15.12.2023 KR 20230183659
(71) Applicant: Sky Labs Inc., Seongnam-si, Gyeonggi-do 13486 (KR)
(72) Inventor: CHANG, Min Soo, Yongin-si, Gyeonggi-do 17128 (KR); LEE, Byung Hwan, Yongin-si, Gyeonggi-do 16981 (KR)
(74) Representative: Loyer & Abello
(86) International application number: PCT/KR2024/017785
(87) International publication number: WO 2025/127433

(57) **Abstract**

The present disclosure relates to a biometric data monitoring system for use in prevention, diagnosis, and management of diseases. The biometric data monitoring system includes a signal collecting unit configured to obtain a biometric signal from a user and a transmitting unit configured to receive a collected signal from the signal collecting unit and transmit the biometric signal to the user or a third party.

## Description

### Technical Field

The present disclosure relates to a biometric data monitoring system having an alarm function, and more particularly, to a biometric data monitoring system configured to collect biometric data from a wearable device and transmit the collected biometric data and enable the data collected from a user to be used by the user or a third party.

### Background Art

Continuous monitoring of biometric signals and biometric data obtained by processing the biometric signals is useful for preventing, early diagnosing, and managing diseases. To monitor such biometric data, a biometric signal detecting device is needed to continuously measure biometric signals easily with high reliability in everyday life. For example, ring-shape devices for collecting biometric data have been recently developed. The present disclosure provides a system having an alarm function that enables a user or a third party to use biometric data collected from the user for prevention, diagnosis, and management of diseases.

### Disclosure of Invention

### Technical Problem

The present disclosure has been made in response to the aforementioned needs and provides a biometric data monitoring system configured to collect biometric data from a wearable device and transmit the biometric data, and enable data collected from a user to be used by the user or a third party.

### Solution to Problem

According to an embodiment of the present disclosure, a biometric data monitoring system having an alarm function includes a signal collecting unit configured to obtain a biometric signal from a user and a transmitting unit configured to receive a collected signal from the signal collecting unit and transmit the biometric signal to the user or a third party.

The biometric signal may include an electrocardiogram, an oxygen saturation, a blood pressure, a heart rate, or a respiratory rate of the user.

The signal collecting unit may be provided on a ring-type or wristwatch-type device wearable by the user.

The third party may include a specific person or hospital designated by the user.

The signal collecting unit may be a device wearable by the user, and the transmitting unit may be a terminal configured to receive and transmit the biometric signal.

The biometric signal may be a photoplethysmogram (PPG) signal collected from the user, and the transmitting unit may be further configured to transmit the PPG signal or predetermined biometric data generated by processing the PPG signal.

The transmitting unit may include an alarming unit configured to notify the user of abnormality of the biometric signal if the biometric signal falls out of a predetermined condition.

According to another embodiment of the present disclosure, a biometric data monitoring system having an alarm function includes a signal collecting unit configured to obtain a biometric signal from a user and a transmitting unit configured to receive a collected signal from the signal collecting unit and notify the user or a third party of abnormality of the biometric signal if the biometric signal falls out of a predetermined condition.

Herein, the biometric signal may include an electrocardiogram, an oxygen saturation, a blood pressure, a heart rate, or a respiratory rate of the user.

Herein, the signal collecting unit may be a ring-type or a wristwatch-type device wearable by the user, and the transmitting unit may be a terminal configured to receive and transmit the biometric signal.

Herein, the third party may include a specific person or hospital designated by the user.

Herein, the biometric signal may be a photoplethysmogram (PPG) signal collected from the user, and the transmitting unit may be further configured to transmit the PPG signal or predetermined biometric data generated by processing the PPG signal.

### Advantageous Effects of Invention

The biometric data monitoring system according to the present disclosure relates to a system that collects biometric data from a wearable device and transmits the biometric data, and enables data collected from a user to be used by the user or a third party, such that the data may be effectively used to prevent, diagnose, and manage diseases.

### Brief Description of Drawings

FIG. 1 is a perspective view of a biometric signal detecting ring according to an embodiment of the present disclosure.
FIG. 2 is an exploded perspective view of FIG. 1.
FIG. 3 is a block diagram of a biometric data monitoring system according to an embodiment of the present disclosure.

### Mode for the Invention

Hereinafter, various embodiments of the present disclosure will be described in relation to the accompanying drawings. Various embodiments of the present disclosure may be modified and have various embodiments, and specific embodiments are illustrated in the drawings and a related detailed description is provided. However, this is not intended to limit the various embodiments of the present disclosure to specific embodiments, and should be understood to include all changes and/or equivalents or substitutes included in the spirit and technical scope of the various embodiments of the present disclosure. In connection with the description of the drawings, similar reference numbers have been used for similar components.

The term "include", "may include", or the like used in various embodiments of the present disclosure is to indicate the presence of functions, operations, elements, etc., disclosed therein, and does not preclude the presence or addition of one or more functions, operations, elements, etc. Moreover, it should be understood that the term "include", "have", or the like used in various embodiments of the present disclosure is to indicate the presence of features, numbers, steps, operations, elements, parts, or a combination thereof described in the specifications, and does not preclude the presence or addition of one or more other features, numbers, steps, operations, elements, parts, or a combination thereof.

When an element is referred to as being "connected" to or by any other element, it should be understood that the element may be directly connected by the other element, but another new element may also be interposed between the element and the other element. Contrarily, when an element is referred to as being "directly connected" or "directly accessed" to or by any other element, it should be understood that there is no new another element between the element and the other element.

The terms used in various embodiments of the present disclosure are for the purpose of describing the particular embodiments only and are not intended to limit the various embodiments of the present disclosure. Singular forms include plural forms unless apparently indicated otherwise contextually.

All of the terms used herein including technical or scientific terms have the same meanings as those generally understood by those of ordinary skill in the art of various embodiments of the present disclosure, unless they are defined other.

The terms defined in a generally used dictionary should be interpreted as having the same meanings as the contextual meanings of the relevant technology and should not be interpreted as having ideal or exaggerated meanings unless they are clearly defined in the various exemplary embodiments of the present disclosure.

Hereinafter, a preferred embodiment according to the present disclosure will be described in detail with reference to the accompanying drawings.

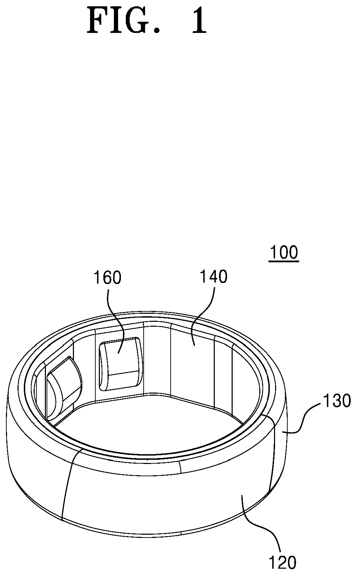

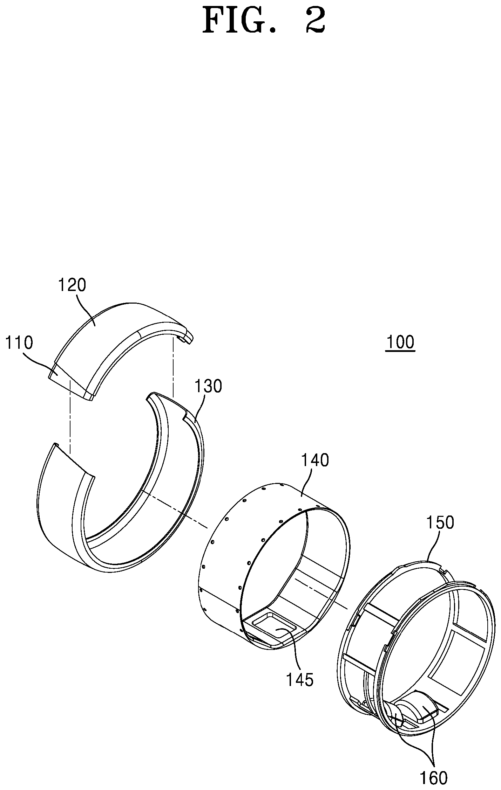

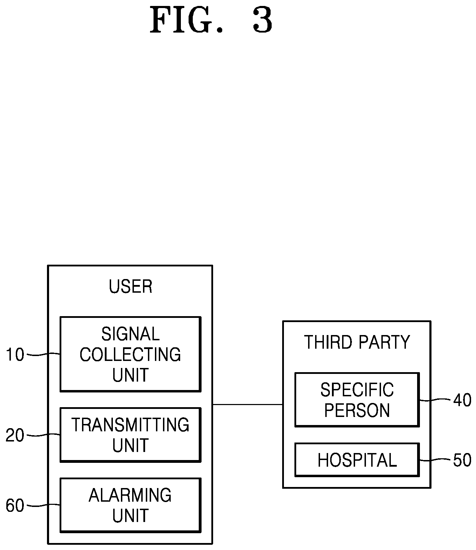

FIG. 1 is a perspective view of a biometric signal detecting ring according to an embodiment of the present disclosure, and FIG. 2 is an exploded perspective view of FIG. 1. FIG. 3 is a block diagram of a biometric data monitoring system according to an embodiment of the present disclosure.

First, the biometric data monitoring system according to an embodiment of the present disclosure may include a signal collecting unit 10 and a transmitting unit 20. The signal collecting unit 10 may be provided to obtain a biometric signal from a user, and the transmitting unit 20 may be provided to receive a collected signal from the signal collecting unit 10 and to transmit the biometric signal to the user or a third party.

According to the current embodiment, the biometric signal collected by the signal collecting unit 10 may include an electrocardiogram, an oxygen saturation, or a blood pressure. The biometric signal collected by the signal collecting unit 10 may not be limited to the biometric signal listed above, and various types of biometric information may be collected. According to the current embodiment, the signal collecting unit 10 may be provided in a user-wearable device such as a ring-type or a wristwatch-type device.

Referring to FIGS. 1 and 2, the signal collecting unit 10 may be provided in a biometric signal detecting ring 100. The biometric signal detecting ring 100 is designed to acquire the user's biometric signal and may include an external electrode 130, an internal electrode 140, an insulating unit 150, a top cover 120, an operation indication unit, and a plurality of sensors 160.

The external electrode 130 may have an arc shape with a partial open section. The external electrode may include a conductor and may function as an electrode that comes into contact with a body of the user to measure an electrocardiogram (ECG). The external electrode may form the exterior of the biometric signal detecting ring 100.

The internal electrode 140 may have a ring shape, and a plurality of openings 145 may be formed to install the plurality of sensors 160 therein. The internal electrode 140 may include a conductor and may function as an electrode for measuring an electrocardiogram. In addition, the internal electrode 140 may form an inner ring portion of the biometric signal detecting ring 100, and the internal electrode 140 may come into contact with a finger of the user.

The insulating unit 150 may be arranged between the external electrode 130 and the internal electrode 140. The insulating unit 150 may enable electrical insulation between the external electrode 130 and the internal electrode 140.

The top cover 120 may have an arc shape so as to close a portion of the external electrode 130 and have the same curvature as that of the external electrode 130 to form a ring together with the external electrode 130. The top cover 120 may be coupled to a portion between the top cover 120 and the external electrode 130 to form the exterior of the biometric signal detecting ring 100.

The plurality of sensors 160 may be arranged to protrude inward from an inner circumferential surface of the internal electrode 140 so as to come into contact with the user's finger. The plurality of sensors 160 may be arranged in each opening 145 of the internal electrode 140. The plurality of sensors 160 may collect a photoplethysmogram (PPG) signal from the user at different positions. The biometric signal detecting ring 100 may further include an accelerometer positioned between the external electrode 130 and the internal electrode 140. The accelerometer may detect movement of the user. In the current embodiment, the accelerometer may detect the movement of the finger of the user.

According to the current embodiment, the signal collecting unit 10 may be provided in the biometric signal detecting ring 100 to collect a biometric signal of the user, but the signal collecting unit 10 may be provided in a user-wearable device. For example, the signal collecting unit 10 may be implemented as a band-type or clock-type device worn on the wrist or ankle, or may be provided on clothing.

The transmitting unit 20 may transmit the biometric signal collected by the signal collecting unit 10 to the user or the third party. According to an embodiment of the present disclosure, the transmitting unit 20 may be a terminal that receives the biometric signal from the biometric signal detecting ring 100 and transmits the biometric signal. The terminal may include, for example, a smartphone or a tablet personal computer (PC). The terminal may be connected to the biometric signal detecting ring 100 wiredly or wirelessly. For example, the terminal may be connected to the biometric signal detecting ring 100 via Bluetooth or Wi-Fi. The transmitting unit 20 may be provided in the terminal to transmit the biometric signal to the user or the biometric signal to the third party via the terminal. The terminal may be connected to the server 30 wiredly or wirelessly. The biometric signal may be stored in the server 30. For example, the terminal may be connected to the server 30 through a mobile telecommunication technology such as 3G, LTE, or 5G. The transmitting unit 20 may transmit the PPG signal collected by the biometric signal detecting ring 100. The transmitting unit 20 may transmit the PPG signal, or transmit predetermined biometric data generated by processing the processed PPG signal. The transmitting unit 20 may be configured together with the signal collecting unit 10 in the biometric signal detecting ring 100 to transmit the biometric signal collected by the signal collecting unit 10 to an external device.

According to the current embodiment, the third party may include a specific person 40 or hospital 50 designated by the user. For example, the user may designate the specific person 40 as the third party, such as a relative, a caregiver, or a primary care physician. The user's biometric signal may be transmitted directly to a person designated as the specific person 40, thereby obtaining user's health information for the biometric signal of the user. The user may designate the third party via the terminal to designated the third party as a recipient of the biometric signal. The third party may be selected by designating, changing, or adding the terminal of the specific person 40 designated by the user.

If the hospital 50 is designated as the third party, the biometric signal may be transmitted to a server of the hospital, allowing medical information related to the user to be updated. The hospital 50 may share the biometric information with the primary care physician of the user in which the biometric information may be used to prevent, diagnose, and manage diseases of the user.

According to the current embodiment, the transmitting unit 20 may further include an alarming unit 60 that notifies abnormality of the biometric signal of the user if the biometric signal falls out of a predetermined condition. For example, if the PPG signal falls outside a predetermined normal range such that the PPG signal is determined as a signal indicating a situation requiring observation or an irregular pulse wave, the alarming unit 60 may notify the user or the third party of an alarm. Furthermore, if a value of an oxygen saturation, a heart rate, a blood pressure, or a blood sugar level as the biometric signal falls out of a predetermined proper range, the alarming unit 60 may transmit an abnormal signal to the user or the third party. The user may specify the predetermined proper range via the terminal.

In addition, a doctor may set the proper range of the biometric signal using the terminal connected to the server of the hospital. The alarming unit 60 may be configured to perform a prevention function by adopting a normal range recommended in the general medical field or conservatively setting a lower range than the normal range by the user. For example, if a systolic blood pressure exceeding 140 mmHg or less than 90 mmHg or a diastolic blood pressure exceeding 90 mmHg or less than 60 mmHg continues for 10 minutes or longer, the alarming unit 60 may transmit an alarm to the user or the third party. In other words, an alarm may be transmitted to at least one of a terminal of the user or the third party. In addition, for example, if an oxygen saturation less than 90 % continues for 10 minutes or longer, the alarming unit 60 may transmit an alarm to the user or the third party. In other words, the alarm may be transmitted to at least one of a terminal of the user or the third party. Through the transmission from the alarming unit 60, the user may directly determine whether the biometric data is abnormal, and the hospital 50 may receive the abnormal signal and use, in real time or retrospectively, data of biometric information in which abnormality is detected for prevention, diagnosis, and management of diseases.

Meanwhile, the biometric data monitoring system having an alarm function according to another embodiment of the present disclosure may include the signal collecting unit 10 that obtains a biometric signal from the user and the transmitting unit 20 that receives a collected signal from the signal collecting unit 10 and notifies abnormality of the biometric signal to the user or the third party if the biometric signal falls outside the predetermined condition.

According to the current embodiment, the signal collecting unit 10 may be provided on the biometric signal detecting ring 100 to collect the biometric signal of the user, as in the embodiment. However, the signal collecting unit 10 may be provided in a user-wearable device, and may be implemented as a band-type or watch-type device worn on the wrist or ankle or may be provided in clothing.

The transmitting unit 20 may receive the biometric signal collected by the signal collecting unit 10 and may transmit the abnormal biometric signal to the user or the third party if the biometric signal deviates from the predetermined condition. The biometric signal may include an electrocardiogram, a heart rate, an oxygen saturation, a blood pressure, a blood sugar level, a pulse rate, or a respiratory rate of the user.

If the biometric signal deviates from the predetermined condition, it may mean that the biometric signal falls outside the predetermined normal range which may be set to a normal range recommended in the general medical field. The user may specify the predetermined condition via the terminal. In addition, a doctor may set the proper range of the biometric signal using the terminal connected to the server of the hospital.

The abnormal biometric signal may be a notification signal indicating abnormality of the biometric signal if the biometric signal deviates from a predetermined range. For example, if a systolic blood pressure exceeding 140 mmHg or less than 90 mmHg or a diastolic blood pressure exceeding 90 mmHg or less than 60 mmHg continues for 10 minutes or longer, the transmitting unit 20 may transmit an abnormal blood pressure signal to the user or the third party. In other words, the abnormal blood pressure signal may be transmitted to at least one of a terminal of the user or the third party. In addition, for example, if an oxygen saturation less than 90 % continues for 10 minutes or longer, the transmitting unit 20 may transmit an abnormal oxygen saturation signal to the user or the third party. In other words, an alarm may be transmitted to at least one of a terminal of the user or the third party. Through the transmission from the transmitting unit 20, the user may directly determine whether the biometric data is abnormal, and the hospital 50 may receive the abnormal signal and use, in real time or retrospectively, data of biometric information in which abnormality is detected for prevention, diagnosis, and management of diseases.

According to an embodiment of the present disclosure, the transmitting unit 20 may be a terminal that receives the biometric signal from the biometric signal ring 100 and transmits the biometric signal. The terminal may include, for example, a smartphone or a tablet PC. The terminal may be connected to the biometric signal detecting ring 100 wiredly or wirelessly. For example, the terminal may be connected to the biometric signal detecting ring 100 via Bluetooth or Wi-Fi. The transmitting unit 20 may be provided in the terminal to transmit the biometric signal to the user or the biometric signal to the third party via the terminal. The terminal may be connected to the server 30 wiredly or wirelessly. For example, the terminal may be connected to the server 30 through a mobile telecommunication technology such as 3G, LTE, or 5G. The transmitting unit 20 may be configured together with the signal collecting unit 10 in the biometric signal detecting ring 100 to transmit the biometric signal collected by the signal collecting unit 10 to an external device.

The transmitting unit 20 may transmit the PPG signal collected by the biometric signal detecting ring 100. The transmitting unit 20 may transmit the PPG signal, or transmit predetermined biometric data generated by processing the processed PPG signal.

According to the current embodiment, the third party may include a specific person 40 or hospital 50 designated by the user. For example, the user may designate the specific person 40 as the third party, such as a relative, a caregiver, or a primary care physician. The user's biometric signal may be transmitted directly to a person designated as the specific person 40, thereby obtaining user's health information for the biometric signal of the user. The user may designate the third party via the terminal to designated the third party as a recipient of the biometric signal. The third party may be selected by designating, changing, or adding the terminal of the specific person 40 designated by the user. If the hospital 50 is designated as the third party, the biometric signal may be transmitted to a server of the hospital, allowing medical information related to the user to be updated. The hospital 50 may share the biometric information with the primary care physician of the user in which the biometric information may be used to prevent, diagnose, and manage diseases of the user.

As such, the biometric data monitoring system having an alarm function according to an embodiment of the present disclosure may collect biometric data from a wearable device and transmit the biometric data to a user or a third party to check a health state in real time based on a biometric signal of the user, and enable the third party to provide emergency assistance upon detection of a danger signal. In addition, the biometric data may be transmitted to a hospital and effectively used for disease prevention, diagnosis, and management.

So far, the present disclosure has been described in detail with preferred embodiments, but the present disclosure is not limited to the above embodiments, and various modifications may be made without departing from the scope of the present disclosure.

## Claims

1. A biometric data monitoring system having an alarm function, the biometric data monitoring system comprising:
a signal collecting unit configured to obtain a biometric signal from a user; and
a transmitting unit configured to receive a collected signal from the signal collecting unit and transmit the biometric signal to the user or a third party.

2. The biometric data monitoring system of claim 1, wherein the biometric signal comprises an electrocardiogram, an oxygen saturation, a blood pressure, a heart rate, or a respiratory rate of the user.

3. The biometric data monitoring system of claim 1, wherein the signal collecting unit is provided on a ring-type or wristwatch-type device wearable by the user.

4. The biometric data monitoring system of claim 1, wherein the third party comprises a specific person or hospital designated by the user.

5. The biometric data monitoring system of claim 1, wherein the signal collecting unit is a device wearable by the user, and
the transmitting unit is a terminal configured to receive and transmit the biometric signal.

6. The biometric data monitoring system of claim 1, wherein the biometric signal is a photoplethysmogram (PPG) signal collected from the user, and the transmitting unit is further configured to transmit the PPG signal or predetermined biometric data generated by processing the PPG signal.

7. The biometric data monitoring system of claim 1, wherein the transmitting unit comprises an alarming unit configured to notify the user of abnormality of the biometric signal when the biometric signal deviates from a predetermined condition.

8. A biometric data monitoring system having an alarm function, the biometric data monitoring system comprising:
a signal collecting unit configured to obtain a biometric signal from a user; and
a transmitting unit configured to receive a collected signal from the signal collecting unit and notify the user or a third party of abnormality of the biometric signal if the biometric signal falls out of a predetermined condition.

9. The biometric data monitoring system of claim 8, wherein the biometric signal comprises an electrocardiogram, an oxygen saturation, a blood pressure, a heart rate, or a respiratory rate of the user.

10. The biometric data monitoring system of claim 8, wherein the signal collecting unit is provided on a ring-type or wristwatch-type wearable device wearable by the user, and
the transmitting unit is a terminal configured to receive and transmit the biometric signal.

11. The biometric data monitoring system of claim 8, wherein the third party comprises a specific person or hospital designated by the user.

12. The biometric data monitoring system of claim 8, wherein the biometric signal is a photoplethysmogram (PPG) signal collected from the user, and the transmitting unit is further configured to transmit the PPG signal or predetermined biometric data generated by processing the PPG signal.
